(19) Europäisches Patentamt
European Patent Office
Office européen des brevets

(11) Veröffentlichungsnummer: **0 378 768 B1**

(12) **EUROPÄISCHE PATENTSCHRIFT**

(45) Veröffentlichungstag der Patentschrift: **11.08.93**

(51) Int. Cl.5: **C07C 51/58**, C07C 59/135

(21) Anmeldenummer: **89120270.7**

(22) Anmeldetag: **02.11.89**

# (54) Verfahren zur kontinuierlichen Oligomerisation von Hexafluorpropenoxid.

(30) Priorität: **14.01.89 DE 3901001**

(43) Veröffentlichungstag der Anmeldung:
**25.07.90 Patentblatt 90/30**

(45) Bekanntmachung des Hinweises auf die Patenterteilung:
**11.08.93 Patentblatt 93/32**

(84) Benannte Vertragsstaaten:
**BE CH DE ES FR GB IT LI NL**

(56) Entgegenhaltungen:
**EP-A- 0 021 221**
**EP-A- 0 315 908**

(73) Patentinhaber: **HOECHST AKTIENGESELLSCHAFT**

**W-6230 Frankfurt am Main 80(DE)**

(72) Erfinder: **Finke, Manfred, Dr.**
**Behringstrasse 25**
**W-6233 Kelkheim (Taunus)(DE)**
Erfinder: **Siegemund, Günter, Dr.**
**Frankfurter Strasse 21**
**W-6238 Hofheim am Taunus(DE)**
Erfinder: **Strutz, Heinz, Dr.**
**Johannesallee 14**
**W-6230 Frankfurt am Main 80(DE)**

EP 0 378 768 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur kontinuierlichen Herstellung von Oligomeren des Hexafluorpropenoxids (HFPO).

Die katalysierte Oligomerisation von HFPO ist bekannt [Angew. Chem. (1985) 97 , 164]. Sie wird diskontinuierlich im Rührkessel bzw. Rührautoklav durchgeführt, wobei HFPO unter Rühren eingeleitet wird, bis ein bestimmter Füllstand im Rührgefäß erreicht ist. Danach ist eine Ruhezeit, gegebenenfalls unter Abkühlung des Reaktionsgemischs, notwendig, um eine Phasentrennung der leichten Katalysatorphase und der schweren Produktphase zu ermöglichen. Eine exakte Phasentrennung ist einerseits notwendig, um möglichst wenig des oft teuren Katalysators mit dem Produkt auszutragen, andererseits sollten die oligomeren Säurefluoride möglichst wenig mit dem Katalysatorgemisch oder Teilen davon kontaminiert sein, um möglichen Problemen bei sich anschließenden Reaktionen bzw. Verwendung der HFPO-Oligomere aus dem Weg zu gehen. Die bislang geübte Technik der diskontinuierlichen Fahrweise ist sehr umständlich und personalintensiv, sie führt infolge der notwendigen Ruhezeit und des oft nur ungenügend ausnutzbaren Reaktorvolumens zu kleinen Raum-Zeit-Ausbeuten und durch den zwangsläufig über weite Strecken nichtstationären Zustand zu Schwankungen in der Produktqualität.

Die Nachteile können behoben werden, indem die Oligomerisation von HFPO kontinuierlich in solchen Fluid-Kontaktierapparaten durchgeführt wird, die alleine oder in Kombination mit zusätzlichen Apparaten eine Abtrennung der schweren Produktphase von der Katalysatorphase während der Reaktion erlauben.

Gegenstand der Erfindung ist demnach ein kontinuierliches Verfahren zur Herstellung perfluorierter Carbonsäurefluoride der Formel

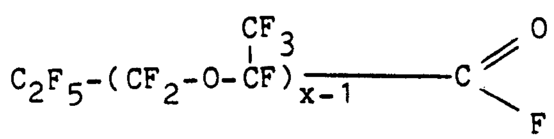

(I)

in der x eine ganze Zahl von 1 bis 31, vorzugsweise 1 bis 8 und insbesondere von 1 bis 5 bedeutet, durch katalysierte Oligomerisation von Hexafluorpropenoxid (HFPO), wobei eine Reaktionsvorrichtung aus einem oder mehreren Reaktionsgefäßen, deren erstes Gefäß mit einer im unteren Drittel seitlich angebrachten Einleitungsvorrichtung, einer Füllstandsregelung und einer am Boden befindlichen Ablaßvorrichtung versehen ist, mit einer Katalysatorlösung beschickt wird, in die über die Einleitungsvorrichtung fortlaufend HFPO bei einer Temperatur von -10 bis +25 °C eingespeist und dabei zu Oligomeren umgesetzt wird, wobei das Gemisch aus Katalysatorlösung und gebildetem HFPO-Oligomeren unterhalb der Einleitungsvorrichtung, oder nach Überführung in ein weiteres Reaktionsgefäß, in Phasen getrennt wird, die Katalysatorphase gegebenenfalls in das Reaktionsgefäß zurückgeführt und die schwerere Produktphase über die am Boden befindliche Ablaßvorrichtung der Reaktionsanordnung entnommen wird.

Derartige Reaktionsvorrichtungen, sogenannte Fluid-Kontaktierapparate sind beispielsweise in Ullmanns Encyklopädie der technischen Chemie, 4. Auflage, Band 3, S. 357 ff., Verlag Chemie 1973 beschrieben.

Einsetzbar sind z.B. Rührreaktoren oder Rührkesselkaskaden, sofern diese mit einem Abscheider verbunden sind und durch reaktionstechnische Maßnahmen gewährleistet wird, daß das Gemisch der Katalysatorphase und der Produktphase in das nächste Reaktionsgefäß bzw. in den Abscheider gelangen. In dem genügend großen Abscheider werden die beiden Phasen getrennt; die Katalysatorphase wird gegebenenfalls in den Rührkessel bzw. die Kaskade zurückgeführt, die Produktphase in ein Vorratsgefäß überführt. Analog sind eine Flüssigkeitsringpumpe oder ein Rohrreaktor einsetzbar.

Bevorzugt werden Gegenstromreaktoren wie beispielsweise Glockenboden- oder Siebbodenreaktoren, Füllkörpersäulen oder sprühturmähnliche Reaktoren. Hier wird die Katalysatorlösung von oben aufgegeben und das HFPO von unten im Gegenstrom geführt. Katalysator- und Produktphase laufen nach unten und werden unterhalb der HFPO-Einspeisung gesammelt und in die Phasen separiert. Die obere Phase (Katalysatorphase) wird abgezogen und wieder am oberen Ende in den Reaktor eingeführt. Die schwere Phase (Produktphase) wird in ein Vorratsgefäß abgelassen.

Das Reaktionsgefäß ist mit einer Füllstandsregelung versehen, die gewährleistet, daß die im unteren Teil befindliche Poduktphase nicht über die Höhe der Einleitungsvorrichtung ansteigt. Auf diese Weise wird sichergestellt, daß das HFPO stets unmittelbar in die Katalysatorphase eingeleitet wird.

Insbesondere bevorzugt werden Fluid-Kontaktierapparate wie Blasensäule, Schlaufenreaktor oder Strahldüsenreaktor, in denen das HFPO vorteilhaft oberhalb der Phasengrenze zwischen Katalysatorlösung und

Produkt in die Katalysatorlösung gasförmig oder flüssig eingeleitet wird; das gebildete Produkt sinkt aufgrund der Schwerkraft durch die Katalysatorphase nach unten und wird unterhalb der Eindosierstelle in einer Ruhezone als eigene Phase separiert und stetig in ein Vorratsgefäß abgelassen. HFPO kann gasförmig oder flüssig eindosiert werden.

Die HFPO-Oligomerisation ist eine exotherme Reaktion. Die auftretende Wärme kann über eine Mantelkühlung, eingebaute Wärmetauscher oder über einen externen Wärmetauscher, durch den die Katalysatorlösung während der Reaktion im Kreis geführt wird, abgeführt werden.

Schließlich ist für den Fall der Herstellung kurzkettiger Oligomerer der Formel (I) (x = 1, 2, 3, 4) auch eine Siedekühlung möglich, indem die kurzkettigen Oligomere (insbesondere wenn x = 1) kontinuierlich über Kopf abgezogen werden.

Gegebenenfalls kann aus einem Vorratsgefäß Katalysatorlösung kontinuierlich oder diskontinuierlich in den eigentlichen Reaktor nachdosiert werden, um ausgetragenen Katalysator zu ersetzen und/oder einen partiellen Katalysatoraustausch während der Reaktion zu ermöglichen.

Als Katalysatoren können bekannte Systeme eingesetzt werden, z.B. Silbernitrat in polaren organischen Lösemitteln (DE-A 20 26 669), $CuCl/CuCl_2$/Acrylnitril/Acetonitril (DE-A 29 24 385), KF/Adiponitril/Acetonitril, CsF/Tetraethylendiglykolether, vorzugsweise aber N,N,N',N'-Tetramethylethylendiamin/Acetonitril allein oder mit Zusatz von CuCl(siehe DE-A 39 01 000.7 vom selben Tage, Titel "Verfahren zur Oligomerisation von Hexafluorpropenoxid"), sofern der Katalysator vorteilhafterweise eine hohe Aktivität und eine lange Standzeit aufweist und eine schnelle und weitgehende Phasentrennung ermöglicht. Eine hohe Aktivität ist deswegen erwünscht, um einen vollständigen Umsatz des eingespeisten HFPO zu gewährleisten.

Die HFPO-Oligomerisation liefert im allgemeinen je nach Katalysatorart und Betriebstemperatur unterschiedliche Oligomerverteilungen. Diese Kriterien haben auch Gültigkeit für die diskontinuierliche Arbeitsweise. Es ist jedoch hervorzuheben, daß das Verfahren gemäß der Erfindung sowohl für die kontinuierliche Oligomerisation von HFPO zu Säurefluoriden der Formel (I) mit dem Selektivitätsschwerpunkt bei (I) (x = 2) als auch für die kontinuierliche Oligomerisation zu höheren Säurefluoriden (I) (x = 3 bis 8) geeignet ist.

Der Vorteil der erfindungsgemäß für die Oligomerisation von HFPO eingesetzten Fluid-Kontaktierapparate besteht darin, daß sie eine kontinuierliche Arbeitsweise ermöglichen, indem sie während der Oligomerisation ein Abscheiden des Oligomerisationproduktes und somit eine niedrige Konzentration an dem gebildeten Produkt in der Katalysatorphase erlauben. Damit ist gewährleistet, daß die Reaktion stets in weitgehender Abwesenheit des gebildeten Produktes abläuft, außerdem ist damit eine erhebliche Zeitersparnis verbunden, so daß kleinere, bzw. einfache Reaktionsapparate einsetzbar sind, die keine beweglichen Teile, z.B. Rührvorrichtungen aufweisen.

Die Arbeitsweise ist einfach und leicht durchführbar und somit weniger personalintensiv. Der quasistationäre Zustand während der kontinuierlichen Oligomerisation bedingt zudem geringere Schwankungen in der Produktqualität.

Höhere HFPO-Oligomere, insbesondere das Trimere, Tetramere und Pentamere des HFPO, finden z.B. als Bausteine für die Herstellung perfluorierter Inertflüssigkeiten Verwendung.

Das Dimere des HFPO dient u.a. als Zwischenprodukt bei der Herstellung von Perfluorpropylvinylether.

## Beispiel

In eine Blasensäule (V4A; Mantelkühlung; Innendurchmesser der Blasensäule = 11 cm) füllt man 6 Liter einer Katalysatorlösung, die aus 5,2 Liter trockenem Acetonitril, 755 ml trockenem N,N,N',N'-Tetramethylethylendiamin und 20 ml Wasser zusammengesetzt ist, unter Schutzgas ein. Bei 10 bis 12 °C werden 6 kg HFPO pro Stunde über eine im unteren Drittel der Säule seitlich angebrachte Sintermetallfritte eingeführt. Die entstehenden HFPO-Oligomeren sammeln sich in einer Ruhezone unterhalb der Eindosierfritte. Wenn die Produktphase, die unterhalb der Eindosierfritte befindliche Füllstandsregulierung erreicht hat, wird die HFPO-Oligomerphase kontinuierlich über ein am unteren Ende des Blasensäulenreaktors befindliches Ventil abgelassen. Nach Erreichen der Regelmarke hat die Katalysatorlösung eine effektive Höhe $h_{eff}$ von 62 cm ($h_{eff}$ = Abstand zwischen Eingasfritte und Katalysatoroberfläche bei Betrieb|. Bei Umsätzen von größer als 99 % wird beispielsweise nach Einleiten von 158 kg HFPO folgende Oligomerverteilung der Verbindung (I) (in Masse-%) gefunden:

| | S Masse-% |
|---|---|
| x = 1 | 15,00 |
| x = 2 | 80,54 |
| x = 3 | 4,46 |

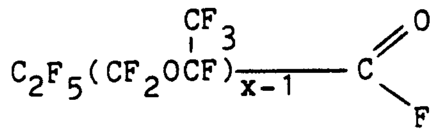

(analysiert als Methylester)

## Patentansprüche

1. Verfahren zur kontinuierlichen Herstellung perfluorierter Carbonsäurefluoride der Formel

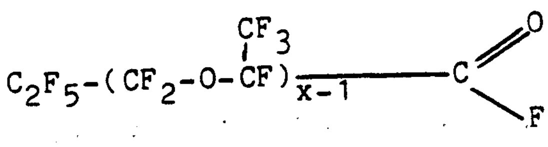

(I)

in der x eine ganze Zahl von 1 bis 31 bedeutet, durch katalysierte Oligomerisation von Hexafluorpropenoxid (HFPO), wobei eine Reaktionsvorrichtung aus einem oder mehreren Reaktionsgefäßen, deren erstes Gefäß mit einer im unteren Drittel seitlich angebrachten Einleitungsvorrichtung, einer Füllstandsregelung und einer am Boden befindlichen Ablaßvorrichtung versehen ist, mit einer Katalysatorlösung beschickt wird, in die über die Einleitungsvorrichtung fortlaufend HFPO bei einer Temperatur von -10 bis +25 °C eingespeist und dabei zu Oligomeren umgesetzt wird, wobei das Gemisch aus Katalysatorlösung und gebildetem HFPO-Oligomeren unterhalb der Einleitungsvorrichtung, oder nach Überführung in ein weiteres Reaktionsgefäß, in Phasen getrennt wird, die Katalysatorphase gegebenenfalls in das Reaktionsgefäß zurückgeführt und die schwerere Produktphase über die am Boden befindliche Ablaßvorrichtung der Reaktionsanordnung entnommen wird.

2. Verfahren nach Anspruch 1, dadurch gekennzeichnet, daß gasförmiges oder flüssiges HFPO eingespeist wird.

3. Verfahren nach Anspruch 1 oder 2, dadurch gekennzeichnet, daß als Reaktionsvorrichtung Gegenstromreaktoren oder Fluid-Kontaktieraggregate eingesetzt werden.

4. Verfahren nach Anspruch 3, dadurch gekennzeichnet, daß Glockenboden-, Siebbodenreaktoren, Füllkörpersäulen, sprühturmähnliche Reaktoren, Blasensäulen, Schlaufenreaktoren oder Strahldüsenreaktoren eingesetzt werden.

5. Verfahren nach einem oder mehreren der Ansprüche 1 bis 4, dadurch gekennzeichnet, daß Katalysatorlösung kontinuierlich oder diskontinuierlich nachdosiert wird.

6. Verfahren nach einem oder mehreren der Ansprüche 1 bis 5, dadurch gekennzeichnet, daß als Katalysatorlösung ein Gemisch aus N,N,N',N'-Tetramethylethylendiamin/Acetonitril allein oder mit Zusatz von CuCl eingesetzt wird.

## Claims

1. A process for the continuous preparation of perfluorinated carbonyl fluorides of the formula

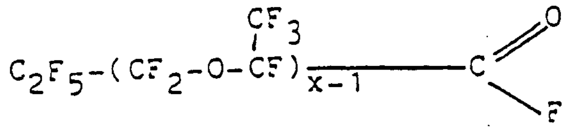

(I)

in which x denotes an integer from 1 to 31, by catalyzed oligomerization of hexafluoropropene oxide (HFPO), entailing a reaction device which is composed of one or more reaction vessels and whose first vessel is equipped with an introduction device which is attached on the side in the lower third, with an appliance for controlling the level of the contents, and with a drainage device located at the bottom, being charged with a catalyst solution into which HFPO is fed continuously at a temperature of -10 to +25 ° C through the introduction device and is thereby converted into oligomers, entailing the mixture of catalyst solution and HFPO oligomers which have formed being separated into phases underneath the introduction device, or after transfer into another reaction vessel, and the catalyst phase optionally being returned into the reaction vessel, and the heavier product phase being removed through the drainage device located at the bottom of the reaction system.

2. The process as claimed in claim 1, wherein gaseous or liquid HFPO is fed in.

3. The process as claimed in claim 1 or 2, wherein countercurrent reactors or fluid-contact units are employed as reaction device.

4. The process as claimed in claim 3, wherein bubble-cap or sieve-plate reactors, packed columns, reactors resembling spray towers, or bubble columns, loop reactors or jet tube reactors are employed.

5. The process as claimed in one or more of claims 1 to 4, wherein catalyst solution is subsequently metered in continuously or discontinuously.

6. The process as claimed in one or more of claims 1 to 5, wherein a mixture of N,N,N',N'-tetramethylethylenediamine/acetonitrile, alone or with added CuCl, is employed as catalyst solution.

## Revendications

1. Procédé de fabrication en continu de fluorures d'acides carboxyliques perfluorés de formule I ci-dessous

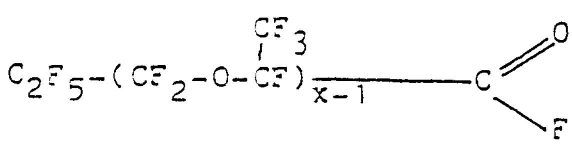

(I)

x étant un entier de 1 à 31, par oligomérisation catalytique d'oxyde d'hexafluoropropène (HFPO) dans un appareil de réaction comportant un ou plusieurs récipients dont le premier, ou le seul, est pourvu d'un dispositif d'introduction placé latéralement à son tiers inférieur, d'un réglage de niveau et d'un dispositif de décharge au fond du récipient, appareil où l'on fait arriver une solution de catalyseur alimentée en continu par le dispositif d'introduction en HFPO à une température de -10 à +25 ° C, lequel est ainsi transformé en oligomère, le mélange de la solution du catalyseur et de l'oligomère formé étant séparé en ses phases au-dessous du dispositif d'introduction, ou bien après l'avoir fait passer dans un autre récipient, on recycle éventuellement la phase du catalyseur au récipient de réaction et on retire la phase de produit plus lourde de l'appareil par le dispositif de décharge du fond.

2. Procédé selon la revendication 1, caractérisé en ce que l'on opère avec du HFPO gazeux ou liquide.

**3.** Procédé selon la revendication 1 ou 2, caractérisé en ce que l'appareil de réaction est un réacteur à contre courant ou une installation pour la mise en contact de fluides.

**4.** Procédé selon la revendication 3, caractérisé en ce que l'on opère dans des réacteurs à plateaux à cloches ou à plateaux perforés, dans des colonnes à corps de garnissage, dans des réacteurs du genre tours de pulvérisation, dans des colonnes d'insufflation, ou encore dans des réacteurs à colonne à bulles à écoulement en boucles ou à buses de jets.

**5.** Procédé selon l'une ou plusieurs des revendications 1 à 4, caractérisé en ce que l'on fait arriver progressivement la solution du catalyseur en continu ou en discontinu.

**6.** Procédé selon l'une ou plusieurs des revendications 1 à 5, caractérisé en ce que la solution du catalyseur est un mélange de N,N,N',N'-tétraméthyléthylène-diamine et d'acétonitrile, seul ou avec du chlorure cuivreux CuCl.